# EUROPEAN PATENT APPLICATION

(11) **EP 2 436 410 A1**
(43) Date of publication of application: **04.04.2012**
(21) Application number: 11194517.6
(22) Date of filing: 28.09.2005
(51) Int. Cl.: A61M 5/00, A61K 49/18, A61M 5/31, A61K 51/12

(54) **Container with constrained quality maintenance agent**

(30) Priority: 28.09.2004 US 613907 P
(62) Divisional of application: 05810234.4
(71) Applicant: Mallinckrodt LLC, Hazelwood, MO 63042 (US)
(72) Inventor: Periasamy, Muthunadar, P., Chesterfield, MO Missouri 63017 (US); Hagen, William, A., O'Fallon, MO Missouri 63366 (US); Mahoney, Michael, R., Wentzville, MO Missouri 63385 (US)
(74) Representative: Cockerton, Bruce Roger

(57) **Abstract**

A container having a quality maintenance additive which maintains the quality of a quantity of liquid or other flowable substance held in the container. The quality maintenance additive is associated with the container so that the quality maintenance additive remains with the container when the liquid or other flowable substance is emptied from the container.

## Description

### BACKGROUND OF THE INVENTION

This invention relates generally to storage containers and the storage of substances, and more particularly to a container for holding substances and maintaining their quality.

The present invention has application in the medical field for medicine and drug storage containers. Particularly, the present invention can be applied to injectable therapeutic or diagnostic drug storage containers, including intravenous injectable drug containers. A more particularly useful application is for diagnostic contrast media containers.

Liquids and other substances which are packaged, shipped and often stored have to be capable of remaining in an efficacious condition for extended periods of time. Almost always this quality or "shelf-life" of the substance is facilitated by the packaging itself, which may protect the substance from contact with air or exposure to sunlight, for example. In these instances, the pertinent properties of the packaging pertain to its ability to form a barrier. The substance being stored may itself contain quality maintenance components. As an example in the case of injectable therapeutic or diagnostic drug substances, the formulation itself may contain components which maintain the quality of the substance. The maintenance of "quality" or "a quality characteristic" of a substance may refer to, among other things, as the maintenance of stability, product attributes and sterility. For example, quality maintenance additives may fall into the class of antimicrobials (preservatives), antioxidants, buffering agents, and/or metal chelating agents (including calcium sequestering agents). Other examples of quality maintenance additives are given in JAMES C. BOYLAN ET AL., PARENTERAL PRODUCTS, in MODERN PHARMACEUTICS (Gilbert S. Banker et al. eds., 3rd ed., 1996), Chapter 12, pp. 441-460, 470, 471, 473-482, and 486-487, incorporated herein by reference. Pages 452-458 of MODERN PHARMACEUTICS particularly discloses added substances for use in parenteral formulations.

The addition of quality maintenance additives to the substance itself requires that the additives not deleteriously affect the use of the substance. In the case of foods or medical products, components added must have minimal or no physiological effect when ingested, injected or otherwise introduced into the living subject.

As one example, contrast media used for imaging the body must be stored while maintaining its efficacy and quality for extended periods. Contrast media may be used for radiographic imaging, magnetic resonance imaging, ultrasound imaging, optical imaging and/or nuclear imaging. Examples of such contrast media are Optiray® X-ray contrast media and OptiMARKⓇ MRI contrast media available from Tyco Health care/Mallinckrodt of St. Louis, Missouri. The contrast media is injected into the patient who is then subjected to an imaging procedure, the contrast media provides a greater contrast so that the image produced of the tissue in the interior of the patient's body is clearer and sharper.

For instance, magnetic resonance imaging contrast media frequently includes a chelated paramagnetic metal ion in a complex, the most common metal ion being gadolinium (Gd⁺³) . Free metal ions are highly undesirable in a living system such as the human body. The contrast media must not have a concentration of free gadolinium metal ions that causes a significant physiological effect on the patient. In addition, it is possible for other metal ions (e.g. , Fe⁺³, Pb⁺²) to be inadvertently introduced into the contrast media during the pharmaceutical manufacturing process.

Manufacturing techniques are employed to minimize the introduction of unwanted metal ions. One step that is commonly taken is to add slight excesses of sodium/calcium salts of chelating agents (e.g., EDTA, DTPA, DTPA-BMEA, "versetamide") to the contrast media to bind free ions of any metal ions introduced in manufacture and metal ions which may come out of their complex. The chelating agent can in some instances maintain the pH of the contrast media within an optimal effective range for extended periods. In some cases, pH of the contrast media may be controlled by the addition of buffering or stabilizing agents. As an example, OPTIRAY® X-ray contrast media contains tromethamine as a buffer. The effective amount of a buffering or chelating agent depends upon the particular application.

In some other cases, the formulation may contain an antioxidant such as ascorbic acid or bisulfite. Of course, the additive is somewhat dependent upon the type of liquid being stored. Whatever the exact consumable or parenteral formulation being stored, where a quality maintenance additive is added, it is unavoidable that some (and usually all) of the agent will be introduced into the body when the product is taken in. It has been suggested in the case of contrast media that if such additives can be avoided, the occurrence of side effects in patients is reduced. See, R. Fattori et al., Iomeprol and Iopamidol in Cardiac Angiography: a Randomised, Double-blind Parallel-group Comparison, Eur. J Radiol. 18 Suppl. 1:S1 - S12 (1994). However, quality maintenance additives are important to providing a product which is stable and of a consistent quality. Therefore, there is a need for a storage and delivery system in which these quality maintenance additives can still be used, but are prevented from entering the patient when the formulation is administered to the patient, or is consumed.

### SUMMARY OF THE INVENTION

In one aspect of the present invention, a container for holding and maintaining quality of a flowable substance generally comprises an enclosure for containing the flowable substance and a quality maintenance additive active to maintain the quality of the flowable substance. The quality maintenance additive is disposed relative to the enclosure so that the quality maintenance additive contacts the flowable substance prior to administration or consumption, such as when held in the enclosure. The quality maintenance additive is capable of acting on the flowable substance to maintain a quality characteristic of the flowable substance prior to administration or consumption, such as when held in the enclosure. The quality maintenance additive is associated with the enclosure so that it remains with the enclosure when the flowable substance is removed from the enclosure for administration or consumption.

In another aspect of the present invention, packaged contrast media generally comprises a quantity of imaging contrast media contained in a syringe barrel, and a plunger head received in the barrel generally adjacent to one end of the barrel. A chelating agent element, as an example of quality maintenance additive, is in contact with the quantity of contrast media in the barrel for capturing free metal ions in the contrast media. The chelating agent is configured to remain with the syringe and not be carried by the contrast media upon ejection of the contrast media from the barrel.

A method of forming a package of a quantity of flowable substance generally comprises associating a quality maintenance additive associated with a container. A quantity of flowable substance is provided which is loaded into the container. The quality maintenance additive is adapted to maintain the quality of the quantity of flowable substance. The association of the quality maintenance additive is such that the quality maintenance additive remains with the container when the flowable substance is emptied therefrom.

Other features of the present invention will be in part apparent and in part pointed out hereinafter.

### BRIEF DESCRIPTION OF THE DRAWINGS

FIG. 1 is a longitudinal section of a syringe prepackaged with a contrast media and with a stem of a syringe plunger disconnected from a plunger head;

FIG. 2 is an enlarged, fragmentary section of a barrel of the syringe taken in the plane including line 2-2 of Fig. 1 and showing a layer of material applied thereto;

FIG. 3 is an enlarged, fragmentary section of the plunger head taken in the plane including line 3-3 of Fig. 1 and showing a layer of material applied thereto;

FIG. 4 is an enlarged, fragmentary longitudinal section of a syringe having chelating agent components mounted thereon;

FIG. 5 is a bottom elevation of a plunger head of a syringe showing a chelating agent component mounted thereon;

FIG. 6 is a section taken in the plane including line 6-6 of Fig. 5;

FIG. 7 is an enlarged, fragmentary section of a syringe similar to Fig. 4 but including a filter at an outlet end of the syringe;

FIG. 8 is a section of a bottle containing contrast media and a floating chelating agent component; and

FIG. 9 is a section of a vial containing contrast media.

Corresponding reference characters indicate corresponding parts throughout the several views of the drawings.

### DETAILED DESCRIPTION OF THE PREFERRED EMBODIMENTS

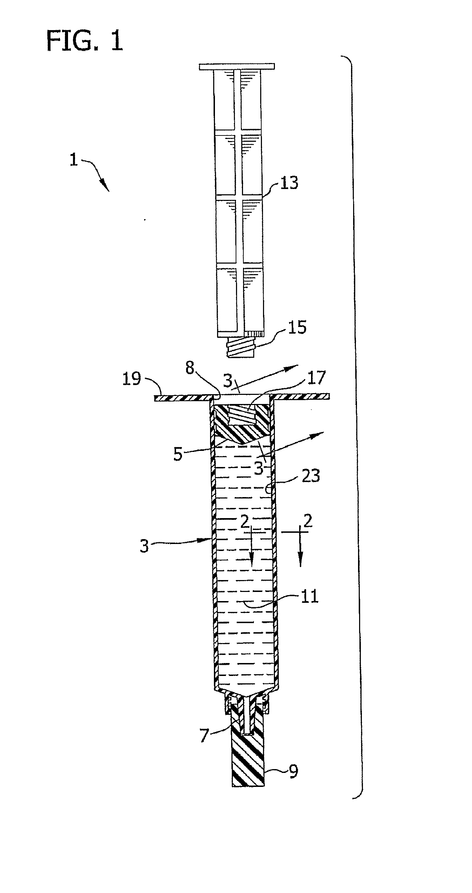

Referring now to the drawings and in particular to Fig. 1, a prefilled syringe (broadly, "a container") is generally indicated at 1. The syringe includes a barrel (generally indicated at 3), a plunger head 5 received in the barrel. The barrel 3 (broadly, "an enclosure") tapers to a nozzle 7 at a constricted end of the barrel. The plunger head is located near an open end 8 of the barrel 3, closing the barrel at that end. The nozzle 7 receives a cap 9 which sealingly closes the nozzle, but can be removed to open the nozzle. A liquid, such as a diagnostic contrast media 11 which may be used for radiographic, magnetic resonance or ultrasound imaging, is contained in the barrel 3 between the cap 9 and the plunger head 5. A plunger stem 13, shown separated from the plunger head 5 in Fig. 1, can be connected in a suitable manner, as by mating threads (designated 15 and 17, respectively), to the plunger head. The plunger so formed can be used to drive the plunger head 5 down within the barrel 3 to push the contrast media 11 through the nozzle 7 and out of the barrel at the time the contrast media is to be injected. The barrel 3 is formed in a conventional way with a flange 19 to facilitate driving the plunger down into the barrel. In the first embodiment illustrated in Fig. 1, the plunger stem 13 would be initially disconnected from the plunger head 5 but packaged together with the assembled barrel 3, contrast media 11, plunger head and cap 9. An example of a syringe of the same general type as described is shown in U.S. Patent No. 6,648,860, the disclosure of which is incorporated herein by reference.

It will be appreciated that, except as described hereinafter, the construction of the syringe 1 can be conventional. However, the present invention is not limited to syringes. A container may be a structure such as a syringe, vial, flask, bottle or jar, pharmacy bulk package or a flexible structure like a bag. The container may be made of glass, plastic, metal, a combination of glass, plastic and/or metal or other suitable materials and combinations of materials. Moreover, the broader term "container" may be taken to mean for purposes of this application structures such as a passage which would contain liquid or other flowable substance only for an instant as it flows through the structure. "Flowable substance," as used herein, includes liquids, emulsions, suspensions and liposomes. The present invention has particular application to containers holding contrast media, and the description will be directed to contrast media 11. However, the present invention is useful with any substance held in a container that can be beneficially affected by a quality maintenance additive which is associated with the container and not directly added to the substance itself in such a way that it is ejected or emptied with the substance from the container in use.

Examples of some types of quality maintenance additives to which the present invention applies are given above in the Background of the Invention. Chelating agents and buffering agents are the most common quality maintenance additives used for contrast media 11, and the embodiments described herein will reference a chelating agent. It will be understood that although generally these additives act on the stored substance to maintain the quality of the stored substance, it is envisioned that a "quality maintenance additive" could also improve or change the quality of the stored substance in some predetermined fashion within the scope of the present invention. Often, the quality maintenance additive acts on the stored substance to remediate a condition which deviates from a desired quality characteristic of the stored substance. A quality characteristic may be, for example and without limitation, a certain pH range, the absence of microbes, the absence of positive metal ions, and prevention of oxidation. Moreover, the present invention is envisioned as having application outside the medical field.

The invention is described herein in terms of an embodiment in which the quality maintenance additive is a chelating agent. In a first embodiment of the present invention, a chelating/stabilizing agent (referred to hereafter as a "chelating agent") is incorporated into a resin that forms the plastic barrel 3 of the syringe 1 using one of the many conventional methods known in the art. Typically, the syringe barrel 3 is made from a polymer or resin material such as polyethylene, polypropylene, a copolymer of polyolefins, polyvinylchloride, polystyrene, polycarbonate, polyethylene terephthalate, cyclic olefin materials and like commonly used polymers. The container can also be made of glass or other suitable materials. One common way of producing the syringe barrel 3 begins with a polymer produced by a basic polymerization reaction (e.g., polypropylene) to which is added, for example, an antioxidant, fiber material (for strength), colorant, nucleators (to improve stiffness and clarity), and possibly a lubricant. Optionally a chelating agent may be added to this mixture. The polymer may take the form of a powder, pellets or other suitable form. The mixture is fed into an extruder which melts the components and extrudes the mixture to form pellets which are sent to a second extruder that injects material into a mold to produce the barrel 3. The chelating agent could be added at any suitable location, such as at either extruder, or between extruders. Heat may be applied to the extruder to facilitate melting. Extrusion polymerization (or reactive extrusion) can be used to functionalize the polymer or to modify existing functional groups on the polymer.

It is further envisioned that a resin already containing a chelating moiety or substituents capable of functioning as chelating agents could be added in place of the chelating agent by itself. Examples of such resin are suitable ion-exchange resins and QuadraPure™ chemical and metal scavengers (functionalized macroporous and microporous resins commercially available from Sigma-Aldrich Corporation). For additional information on resins with such substituents, see "Chapter 3. Ion Exchange" in "Separation and Purification Techniques in Biotechnology" by F. J. Dechow published in 1989 by Noyes Publications, Park Ridge NJ, USA. Other polymers that could be used for this purpose, i.e. crosslinked copolymers of basic vinyl heterocycles with another monomer coploymerizable therewith, are described in U.S. Patent No. 5,094,867, the disclosure of which is incorporated herein by reference.

The chelating agent added to the resin mixture should be able to withstand the various molding process conditions, e.g. shear forces in the extruder and extrusion temperature. The chelating agent would be added in an amount which is sufficient to ensure that in the resin forming the barrel 3, enough of the chelating agent is exposed on an inner wall 23 of the barrel to the contrast media 11 to bind free metal ions in the contrast media. The chelating agent must not affect the strength of the barrel 3 to the extent that it cannot function for its intended purpose, and preferably should also not affect the clarity of the barrel. Moreover other processes for forming the barrel that are known in the art could be used, such as sintering where the material could be hot or cold formed, or co-extrusion where only an internal layer includes the chelating agent and an outer layer(s) maintains desired mechanical properties.

Other versions of this form of the present invention are contemplated. For instance, the head 5 of the plunger, and/or the cap 9 that closes the nozzle 7 could be made from a material including a chelating agent. As another option, chelating agents, such as DTPA or EDTA in the anhydride form, or buffering agents, such as tromethamine, could be attached (e.g., chemically bonded) to a suitable functional group, such as amine, primary amide, carboxylic, hydroxyl or phenolic group already present on the inner side of the container. Examples of such methods are well known in the art. The following recent publications are exemplary: Anal. Chem. 2005, 77, 30-35 and Anal. Chem. 2005, 77, 1096-1105. It is also envisioned that a chelating agent could be added to a mixture for making other containers such as a glass container. It will be appreciated that by associating the chelating agent with the syringe 1 (or other container such as a vial or bottle), the metal ions and chelating agents remain in the syringe after the contrast media 11 has been ejected from the syringe. Thus, neither is injected into the patient.

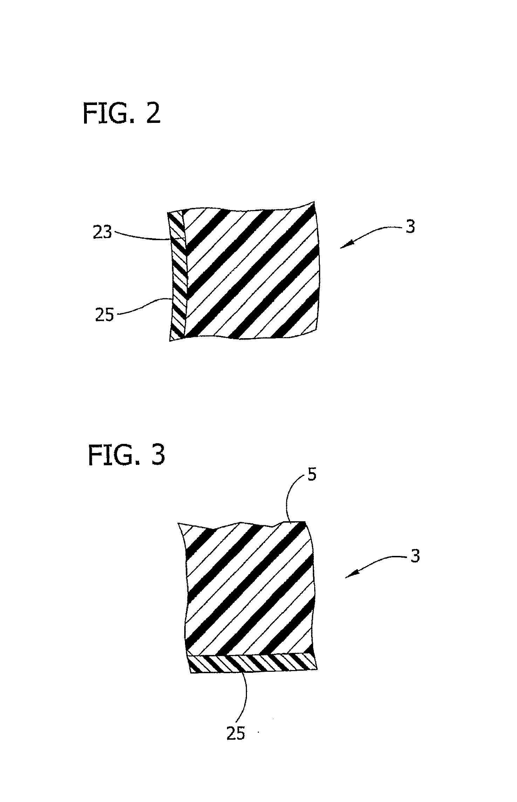

In a second embodiment of the present invention, the quality maintenance additive can be applied to a surface of the container, as an example, syringe 1 (that is exposed to the contrast media 11) after the syringe is formed. It is well known to apply a lubricant, such as silicone to the barrel 3 and/or plunger head 5 to facilitate sliding movement of the plunger head 5 along the wall 23 when used to eject the contrast media 11 from the barrel. The quality maintenance additive, e.g. a chelating agent or a buffering agent, can be part of the silicone (or other lubricant), such as by being chemically attached to the silicone molecule or the other lubricant via a functional group (e.g., an amine, primary amide, carboxylic, hydroxyl or a phenolic group) in the silicone or the other lubricant. Figure 2 illustrates a layer 25 of silicone including a chelating agent on the inner wall 23 of the barrel 3. The silicone and chelating agent layer 25 sticks to the plunger head 5 or the barrel 3 so that as the plunger head moves along the wall toward the nozzle end of the barrel, the silicone and chelating agent are not sheared off into the contrast media 11 in any substantial quantity.

Although possible, typically the composition containing a lubricant and a chelating agent does not chemically bond with the resin material of the barrel 3 when applied thereto. However, optionally, the lubricant with the quality maintenance additive, such as a chelating agent or a buffering agent, could be either chemically or by some other fashion bonded to the surface of the container such as the syringe barrel and/or the plunger. The Gelest Catalog 3000-A titled "Silicon Compounds: Silanes & Silicones" published by Gelest, Inc. in 2004 describes examples of this and other related methods. For other methods of coating surfaces, see "Organic Coatings: Science and Technology", second edition 1999, edited by Z. W. Wicks, Jr., F. N. Jones and S. Peter Pappas and published by Wiley-Interscience.

It will be understood that the chelating agent could be applied without being formulated as part of a lubricant. For instance, if the material of the barrel 3 and/or the plunger head 5 includes a lubricant as a component it would not require additional lubricant applied to the plunger head or the barrel. It is also envisioned that the chelating agent could be attached to a material applied to the barrel 3 and/or head 5 which is not a lubricant. The material would not interfere with the normal operation of the lubricant, but allow the chelating agent to act on the liquid in the barrel 3. Moreover, if the container is not a syringe and does not have a plunger head 5 engaging and moving along an inner wall 23 of the barrel 3, no lubricant is needed. The chelating agent can be formulated in any suitable manner for adhering to the inner wall of the container. Also, physical roughening or etching can be employed to increase the surface area of the inner wall 23, so that the inner wall may then be more readily coated with a material such as a lubricant that is bonded with desired quality maintenance additive(s), such as a chelating agent.

The layer of silicone or other material including a chelating agent (Fig. 2) can be applied in several ways. Conventionally, silicone can be applied by spraying, and could be so applied when formulated with a chelating agent. One type of silicone elastomer believed to be suitable is used to manufacture SILICON® autoclavable silicone tubing, available from NewAge Industries of Southampton, Pennsylvania. In manufacture, the barrels (prior to insertion of the plunger head 5) may be inverted so that the large open end 8 faces down and the nozzle 7 is located at the top. A spray head (not shown) may be inserted into the large open end 8 of the barrel 3 for spraying onto the inner wall 23 of the barrel. A high pressure spray release using a suitable gas such as air or nitrogen as a carrier may be employed. As one alternative, spattering could be used to apply the silicone and chelating agent composition. Spattering can be carried out by vibrating a membrane (not shown) so that it will throw off the material to be deposited. It is also possible to heat the material so that it spatters onto the barrel 3. More generally, heat, such as applied by a high voltage arc, may be applied to break down the material for redepositing on the barrel 3. Still further, the silicone and chelating agent may be applied by dipping the barrel 3 into a liquid form of the material so that a layer is deposited in the barrel. Similarly, the barrel 3 could be tumbled in an emulsion of the chelating agent (or silicone and chelating agent) suspended in water or alcohol (or other suitable emulsifier) to deposit a layer of the chelating agent within the barrel. Other ways of applying the silicone and chelating agent to the barrel 3 that are known to those of ordinary skill in the art also may be employed.

Alternatively, as an example, the chelating agent, as a weak Ca or other metal ion complex, can be coated on a container surface in the form of a polymer ink. See Proc. Intl. Soc. Mag. Reson. Med. 13 (2005) page 2142 for a description of the use of a polymer ink. The following publications describe examples of a methodology for the formation of stable, derivatized film coating on a silica surface: Anal. Chem. 2001, 73, 2429-2436 and Anal. Chem. 2003, 75, 3518-3530.

The chelating agent could also be applied in a conformal coating (vapor deposition) process. A suitable material in dimer form (e.g., parylene, polypropylene), including a chelating agent element is heated to a vapor phase in a vacuum chamber. The barrel 3 of the syringe 1 is also placed in the vacuum chamber. The vapor flows over and is deposited on the entire exposed surface area of the barrel 3 in a highly uniform layer. The deposited material on the barrel 3 attaches to the material of the barrel, forming a strong connection between the deposited material and the barrel. It is also envisioned that other techniques for associating the chelating agent (or other quality maintenance additive) with the barrel 3 could be used, such as nanocoating technology or a biologically active coating technique e.g., using an appropriately derivatized agent of hyaluronan. See, for example, "The chemistry, biology and medical applications of Hyaluronan and its derivatives", ed. T. C. Laurent, Wenner-Gren International Series Vol. 72, Portland Press, London.

Referring to Fig. 3, the layer of material including the chelating agent can also be applied to the plunger head 5. The face of the plunger head does not engage the plunger head 5 of the barrel 3, so that the chelating agent would not need to be part of a lubricant and would not be subject to shear forces. The chelating agent can be applied in the same ways as described above for application of a layer of material to the inner wall 23 of the barrel 3. Moreover, the formulation of the material of the plunger head 5 could include a chelating agent, as described above for the barrel 3.

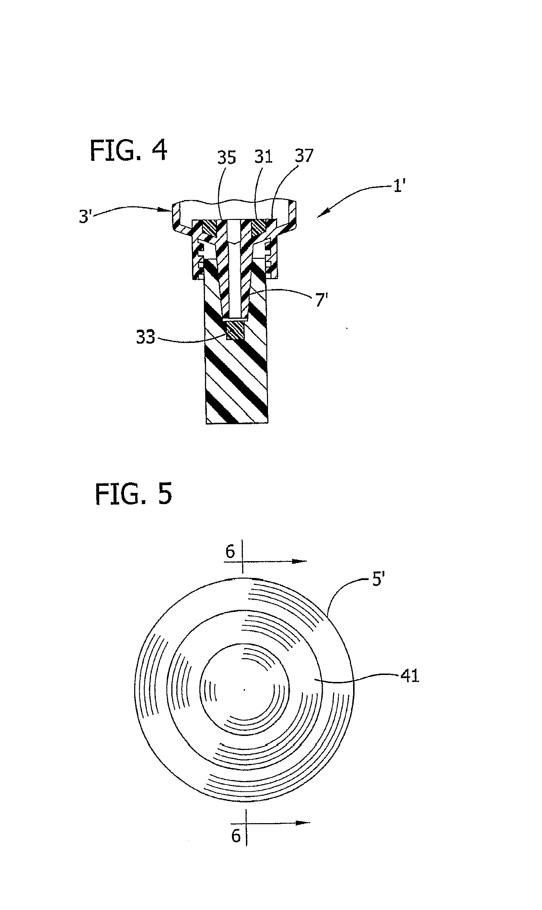

A third embodiment provides a chelating agent that is formed as a separate component and positioned so as to contact the contrast media. Ideally (although not exclusively), these components would be placed in a syringe 1' in areas which are not subject to wear by action of a plunger head 5' moving in a barrel 3'. Corresponding parts of the third embodiment will be labeled with the same reference numerals as for the first and second embodiments, with the addition of a trailing prime. As shown in Fig. 4, the component could be a ring 31 of material including the chelating agent. Alternatively, or in addition to the ring 31 as shown in Fig. 4, a plug 33 of material including a chelating agent may be mounted in a cap 9' so that it is exposed to the contrast media (not shown) at the outlet end of a nozzle 7'. The component material could be porous or nonporous. The advantage of having a porous material would be in the increase of surface area to which the contrast media can be exposed. As one example, the ring 31 and plug 33 could be made of a sintered polypropylene or EPTFE impregnated with a chelating agent, for example, the ion-exchange resin or QuadraPure™ resin. The barrel 3' and ring 31 are formed so that the ring fits in the lower end of the barrel above the nozzle between an inner annular rib 35 and an outer concentric annular rib 37 that are formed in the bottom of the barrel to increase the strength of the barrel. In this location, the ring 31 does not interfere with movement of the plunger head (not shown) and is not subject to wear by engagement with the plunger head.

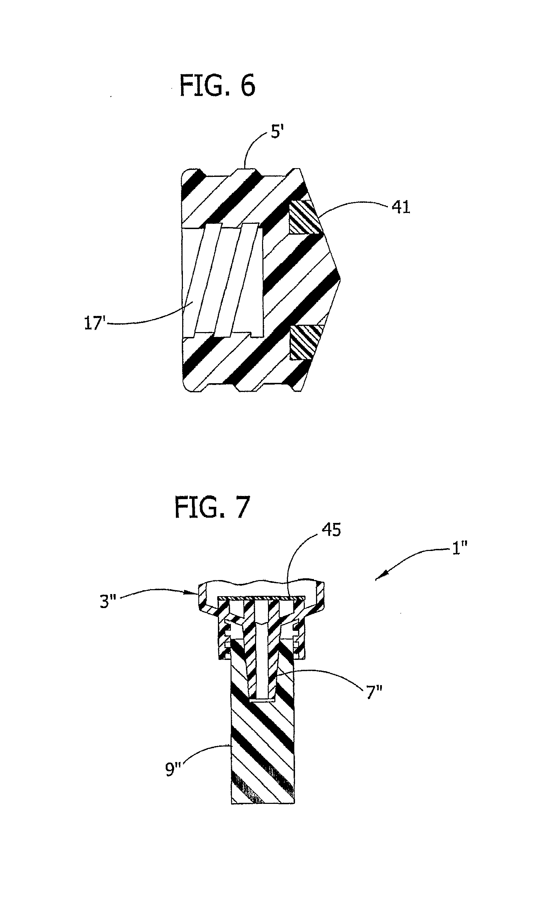

A plunger head 5' in a modified form of the embodiment of Fig. 4 is illustrated in Figs. 5 and 6. A ring 41 including a chelating agent is formed so that it can be mounted within the plunger head 5'. As shown in Fig. 5, the ring 41 faces and is open to the contrast media. In this embodiment, the ring 41 forms part of the surface of the plunger head that pushes the contrast media out of the syringe barrel. The plunger head 5' may be used in conjunction with the ring 31 and/or plug 33 of Fig. 4, or may be used by itself. It will be appreciated that other locations of a chelating component (not shown) within a syringe may be used without departing from the scope of the present invention.

In yet another version of this third embodiment shown in Fig. 7, the chelating agent could be incorporated into filter 45 which is secured to the lower end of a barrel 3'' of a syringe 1'', where the contrast media passes into a nozzle 7''. A filter (not shown) having an incorporated chelating agent could be located away from the barrel 3''. The filter could be positioned at the outlet of the nozzle 7'' or downstream from the outlet before the contrast media is injected into the patient. In such cases, the filter would be sterilized. If the filter (not shown) is away from the syringe, it is still "associated" with the syringe in the sense that it does not move with the contrast media into the patient, but remains with the syringe outside the patient's body. The contrast media from the barrel 3'' can still flow through the filter to remove metals as described for the filter 45. Parts corresponding to those of the syringe 1 are given the same reference numeral, followed by a double prime. Although shown solid, the filter 45 has small pores which would permit passage of the contrast media through the filter. Even further, the chelating agent could be formed as a film which is laminated to the syringe barrel and/or plunger head. It is believed that a laminated film layer would add strength to the barrel, reducing the possibility of syringe cracking during use. If the chelating agent is laminated to the inner wall of the barrel, it would need to be smooth enough to allow the plunger head to slide along the material, and robust enough to avoid being sheared off by the movement of the plunger head. The laminated film layer appears similar to the layer 25 illustrated in Fig. 2.

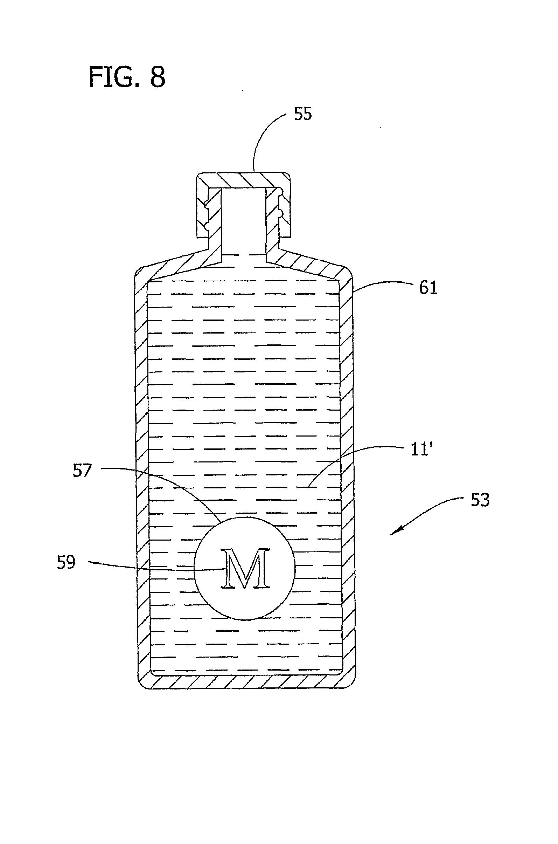

Figure 8 illustrates a container in the form of a bottle (indicated generally at 53) containing a contrast media 11' and including a lid 55. The bottle 53 may be made of a suitable material, such as glass or plastic, by conventional processes known in the art. In this further modification of the embodiment of Fig. 4, the chelating agent component is not attached to or mounted on any part of the bottle 53. The component takes the form of a disk 57 which may float or rest on the bottom of the bottle in the contrast media 11' . Moreover, it is envisioned that the disk 57 could be fixed to the bottle (e.g., at the bottom of the bottle). Still further, the chelating agent component may not have a disk shape. For example, the chelating agent component could take the form of microspheres or beads (spherically or otherwise shaped). For example, the microspheres could be coated with materials containing a quality maintenance additive or the quality maintenance additive attached either chemically or by any other conventional method known in the art. As long as the beads (or other forms of chelating agent component) are large enough not to be taken into a syringe through a needle opening, they need not necessarily be larger than the container opening. Moreover, the beads may float or rest on the bottom of the container. The disk 57 may be formed in a decorative manner, or may be marked with information 59 identifying the maker or type of product. The bottle 53 is formed with a neck 61 optionally having an internal diameter that is smaller than the disk 57 so that the disk is trapped in the bottle when the contrast media 11' is poured out.

The configuration of the bottle 53 and/or disk 57 may be other than described without departing from the scope of the present invention. For example, a container (not shown) may gradually taper in internal diameter from the bottom to the top. As another example, the chelating agent could be formed as beads that are contained in a porous container (e.g. , a mesh bag) that allows the beads to be in liquid contact with the contrast media.

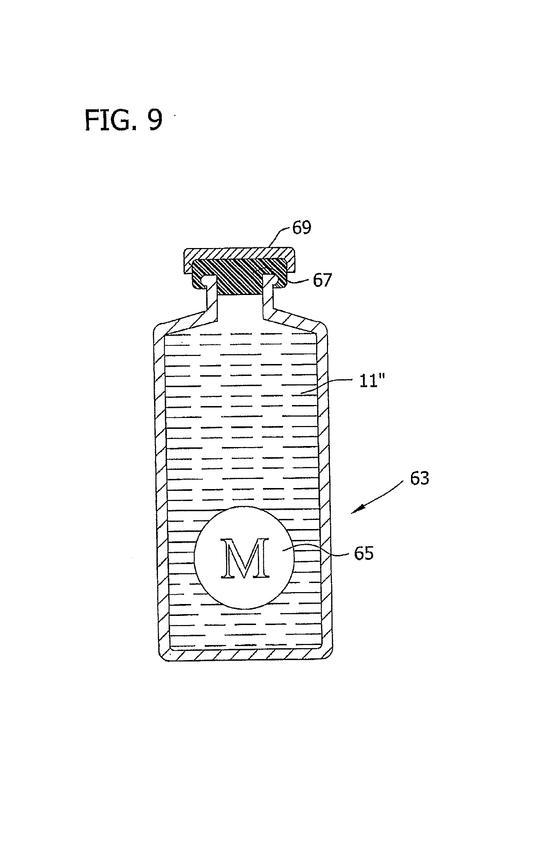

Figure 9 illustrates a version of the present invention in the form of a vial generally indicated at 63. The version is similar to Fig. 8 in that the chelating agent component is a disk 65 that floats or rests on the bottom of the bottle in the contrast media 11". The vial 63 may be made of a suitable material, such as glass or plastic. It will be understood that the chelating agent may be incorporated into the material of the vial 63. The vial includes a puncturable seal 67 covered by a cap piece 69.
The following is a non-exhaustive list of embodiments of the invention which may or may not be claimed.
1. A container for holding and maintaining quality of a flowable substance, the container comprising an enclosure for containing the flowable substance and a qi[alpha]ality maintenance additive active to maintain the quality of the flowable substance, the quality maintenance additive being disposed relative to the enclosure so that the quality maintenance additive contacts the flowable substance when held in the enclosure, the quality maintenance additive being capable of acting on the flowable substance to maintain a quality characteristic of the flowable substance when held in the enclosure, the quality maintenance additive being associated with the enclosure so that it remains with the enclosure when the flowable substance is removed from the enclosure.
2. A container as set forth in embodiment 1 wherein the enclosure constrains the quality maintenance additive from leaving the enclosure.
3. A container as set forth in embodiment 2 wherein the quality maintenance additive is part of a composition of material forming the enclosure.
4. A container as set forth in embodiment 2 wherein the enclosure includes at least one surface disposed for contact with flowable substance held in the container, the quality maintenance additive being disposed on said one surface.
5. A container as set forth in embodiment 4 wherein the quality maintenance additive disposed on said one surface is chemically bonded to the enclosure.
6. A container as set forth in embodiment 4 further comprising another material disposed on said one surface of the enclosure, the quality maintenance additive being in composition with said other material.
7. A container as set forth in embodiment 6 wherein said one surface of the enclosure is etched prior to depositing said other material thereon.
8. A container as set forth in embodiment 7 wherein said other material is a lubricant.
9. A container as set forth in embodiment 2 wherein the quality maintenance additive is formed as a piece separate from the enclosure.
10. A container as set forth in embodiment 9 wherein the quality maintenance additive piece is disposed in the enclosure, the enclosure including an outlet sized for preventing the quality maintenance additive piece from leaving the enclosure.
11. A container as set forth in embodiment 10 in combination with flowable substance contained in the enclosure, the quality maintenance additive piece being at least partially immersed in the flowable substance.
12. A container as set forth in embodiment 9 wherein the quality maintenance additive piece is attached to the enclosure in a position for contact with flowable substance contained in the enclosure.
13. A container as set forth in embodiment 12 wherein the quality maintenance additive piece is a film laminated to the enclosure.
14. A container as set forth in embodiment 9 wherein the quality maintenance additive piece comprises a filter adapted to receive the flowable substance therethrough upon removal of the flowable substance from the enclosure.
15. A container as set forth in embodiment 1 wherein the quality maintenance additive is capable of remediating a quality characteristic of the flowable substance.
16. A container as set forth in embodiment 1 wherein the quality maintenance additive is selected from at least one of an antimicrobial, an antioxidant, a chelating agent or a buffering agent.
17. A container as set forth in embodiment 16 wherein the quality maintenance additive is a chelating agent.
18. A container as set forth in embodiment 17 in combination with the flowable substance, and wherein the flowable substance comprises a contrast media.
19. A container as set forth in embodiment 18 wherein the chelating agent piece is floated in the contrast media.
20. Packaged contrast media comprising: a quantity of imaging contrast media; a container containing the quantity of the contrast media therein; a quality maintenance agent in contact with the quantity of contrast media for maintaining the quality of the contrast media, the quality maintenance agent being configured to remain with the container and not being carried by the contrast media upon removal of the contrast media from the said container.
21. Packaged contrast media as set forth in embodiment 20 wherein, the container is selected from a vial, a bottle, a syringe, a bag or a pharmacy bulk package.
22. Packaged contrast media comprising: a quantity of imaging contrast media; a syringe barrel containing the quantity of the contrast media therein; a plunger head received in the barrel generally adjacent to one end of the barrel; a chelating agent element in contact with the quantity of contrast media in the barrel for capturing free metal ions in the contrast media, the chelating agent being configured to remain with the syringe and not be carried by the contrast media upon ejection of the contrast media from the barrel.
23. Packaged contrast media as set forth in embodiment 22 wherein the chelating agent is associated with at least one of the barrel and the plunger head.
24. Packaged contrast media as set forth in embodiment 23 wherein the chelating agent comprises a layer of material attached to one of the barrel and the plunger head.
25. Packaged contrast media as set forth in embodiment 24 wherein the layer of material includes a lubricant.
26. Packaged contrast media as set forth in embodiment 24 wherein the layer of material is a film laminated to at least one of the barrel and plunger head.
27. Packaged contrast media as set forth in embodiment 23 wherein the contrast agent is incorporated into material forming at least one of the barrel and the plunger head.
28. Packaged contrast media as set forth in embodiment 23 further comprising a piece made from material including a chelating agent, the piece being mounted on one of the barrel and the plunger head.
29. Packaged contrast media as set forth in embodiment 22 further comprising a piece made from material including a chelating agent.
30. Packaged contrast media as set forth in embodiment 29 wherein the chelating agent component comprises a filter arranged to receive contrast media therethrough upon ejection of the contrast media from the barrel.
31. A method of forming a package of a quantity of flowable substance, the method comprising: associating a quality maintenance additive with a container; providing a quantity of flowable substance; loading the quantity of flowable substance into the container, the quality maintenance additive being adapted to maintain the quality of the quantity of flowable substance, the association of the quality maintenance additive being such that the quality maintenance additive remains with the container when the flowable substance is emptied therefrom.
32. A method as set forth in embodiment 31 wherein the step of associating a quality maintenance additive comprises formulating material with a quality maintenance additive and forming the container from the material.
33. A method as set forth in embodiment 31 further comprising the step of depositing a substance including the quality maintenance additive onto a surface of the container which will be exposed to the quantity of flowable substance loaded therein.
34. A method as set forth in embodiment 33 further comprising the step of etching the surface of the enclosure prior to depositing the substance including the quality maintenance additive thereon.
35. A method as set forth in embodiment 34 wherein the step of depositing the substance comprises one of spraying the substance onto the container surface, vapor depositing the substance onto the container surface, and laminating a film including the quality maintenance additive onto the container surface.
36. A method as set forth in embodiment 31 further comprising forming a piece including the quality maintenance additive separately from the container and operatively connecting the piece to the container.
37. A method as set forth in embodiment 36 wherein the step of associating the quality maintenance additive with the container comprises fixing the piece to the container.
38. A method as set forth in embodiment 36 wherein the piece is floated in the quantity of flowable substance.
39. A method as set forth in embodiment 36 wherein the piece rests on the bottom of the container.
40. A container for holding and maintaining quality of a flowable medical substance for use in medical diagnostics and/or treatment, the container comprising an enclosure for containing the flowable medical substance and a quality maintenance additive active to maintain the quality of the flowable medical substance, the quality maintenance additive being disposed relative to the enclosure so that the quality maintenance additive contacts the flowable medical substance when held in the enclosure, the quality maintenance additive being capable of acting on the flowable medical substance to maintain a quality characteristic of the flowable medical substance when held in the enclosure, the quality maintenance additive being associated with the enclosure so that it remains with the enclosure when the flowable medical substance is removed from the enclosure.
When introducing elements of the present invention or the preferred embodiment (s) thereof, the articles "a", "an", "the" and "said" are intended to mean that there are one or more of the elements. The terms "comprising", "including" and "having" are intended to be inclusive and mean that there may be additional elements other than the listed elements. The use of terms indicating a particular orientation (e.g., "top", "bottom", "side", etc.) is for convenience of description and does not require any particular orientation of the item described.
As various changes could be made in the above without departing from the scope of the invention, it is intended that all matter contained in the above description and shown in the accompanying drawings shall be interpreted as illustrative and not in a limiting sense.

## Claims

1. A syringe for holding and maintaining quality of a flowable substance, the syringe comprising a barrel for containing the flowable substance, a plunger head movable within the barrel, a nozzle at a first end of the barrel and a quality maintenance additive active to maintain the quality of the flowable substance, the quality maintenance additive being disposed relative to the barrel so that the quality maintenance additive contacts the flowable substance when held in the barrel, the quality maintenance additive being capable of acting on the flowable substance to maintain a quality characteristic of the flowable substance when held in the barrel, the barrel adapted to constrain the quality maintenance additive from leaving the barrel so that it remains with the barrel when the flowable substance is removed from the barrel, wherein the quality maintenance additive is formed as a piece separate from the barrel; **characterised in that:**
the quality maintenance additive piece is placed in an area which is not subject to wear by action of the plunger head moving within the barrel.

2. A syringe as set forth in claim 1 wherein the quality maintenance additive piece is disposed in the barrel, the barrel including an outlet sized for preventing the quality maintenance additive piece from leaving the enclosure.

3. A syringe as set forth in claim 2 in combination with flowable substance contained in the barrel, the quality maintenance additive piece being at least partially immersed in the flowable substance.

4. A syringe as set forth in claim 1 wherein the quality maintenance additive piece is attached to the barrel in a position for contact with flowable substance contained in the barrel.

5. A syringe as set forth in claim 1 wherein the quality maintenance additive piece comprises a filter adapted to receive the flowable substance therethrough upon removal of the flowable substance from the barrel.

6. A syringe as set forth in claim 1 wherein the quality maintenance additive is capable of remediating a quality characteristic of the flowable substance.

7. A syringe as set forth in claim 1 wherein the quality maintenance additive is selected from at least one of an antimicrobial, an antioxidant, a chelating agent or a buffering agent.

8. A syringe as set forth in claim 7 wherein where the quality maintenance additive is a chelating agent and the barrel is in combination with the flowable substance, the flowable substance comprises a contrast media.

9. A syringe as set forth in claim 8 wherein the quality maintenance additive piece made is floated in the contrast media.

10. A syringe as set forth in any preceding claim, wherein the flowable substance is a flowable medical substance for use in medical diagnostics and/or treatment.

11. A syringe as set forth in any preceding claim, wherein the quality maintenance additive piece is a ring of material which is fitted in the first end of the barrel proximate the nozzle.

12. A syringe as set forth in any of claims 1 to 10, wherein the quality maintenance additive piece is a ring of material which is mounted within the plunger head.

13. Packaged flowable substance comprising:
a quantity of flowable substance;
a syringe barrel according to any preceding claim containing the quantity of the flowable substance therein;
the quality maintenance additive being a chelating agent element for capturing free metal ions in the contrast media when held in the barrel.

14. Packaged flowable substance as set forth in claim 13 further comprising a piece made from material including a chelating agent, the piece being mounted on one of the barrel and the plunger head.

15. Packaged flowable substance as set forth in claim 13 further comprising a piece made from material including a chelating agent.

16. Packaged flowable substance as set forth in claim 15 wherein the chelating agent piece comprises a filter arranged to receive the contrast media therethrough upon ejection of the contrast media from the barrel.
